# EUROPEAN PATENT APPLICATION

(11) **EP 2 239 575 A1**
(43) Date of publication of application: **13.10.2010**
(21) Application number: 08866532.8
(22) Date of filing: 04.12.2008
(51) Int. Cl.: G01N 33/569, G01N 33/53, G01N 33/543, G01N 33/553, C07K 16/10

(54) **REAGENT FOR DETECTING HIV-1 ANTIGEN AND DETECTION METHOD**

(30) Priority: 28.12.2007 JP 2007340370
(71) Applicant: Sysmex Corporation, Kobe-shi, Hyogo 651-0073 (JP)
(72) Inventor: MIYAJI, Miki, Kobe-shi Hyogo 651-0073 (JP); OHKI, Taku, Kobe-shi Hyogo 651-0073 (JP)
(74) Representative: Paemen, Liesbet R.J.
(86) International application number: PCT/JP2008/072068
(87) International publication number: WO 2009/084369

(57) **Abstract**

The present invention provides a reagent for detecting a HIV antigen comprising a first antibody wherein a first human monoclonal antibody recognizing HIV-1 p24 antigen is labeled with a labeling substance, a solid phase and a second antibody wherein a substance capable of binding to the solid phase is bound to a second human monoclonal antibody recognizing HIV-1 p24 antigen.

## Description

### TECHNICAL FIELD

The present invention relates to a reagent and method for detecting HIV-1 p24 antigen.

### BACKGROUND ART

Acquired Immunodeficiency Syndrome (AIDS) is a disease caused by infection with human immunodeficiency virus (HIV). AIDS has been usually diagnosed by detecting HIV antibodies in blood. However, there appears a blank period at early stage of HIV infection when a titer of the HIV antibodies in blood does not increase. Therefore, patients at early stage of HIV infection may be overlooked by the test methods detecting HIV antibodies.

Methods for detecting HIV antigens in blood are known to solve the above-mentioned problem. There are also known methods in which p24 core protein of HIV type 1 virus (hereinafter referred to as "HIV-1 p 24 antigen") that shows stable antigenicity is detected. Such conventional methods include, for example, sandwich immunoassays described in USP 5,173,399, USP 4,983,529, USP 6,432,633 and the like.

### SUMMARY OF INVENTION

### Technical Problem

High sensitivity is required for the detection of HIV antigens in blood because HIV antigens are contained in blood in trace amount. Mouse monoclonal antibodies are used as the antibodies for detecting HIV-1 p24 antigen in the conventional immunoassays. Thus, when a sample contains human anti-mouse antibody (HAMA), the mouse monoclonal antibody contained as a reagent may react with HAMA in the sample to give false positive results.

Accordingly, the present invention is to provide a reagent and method which can decrease false positive results due to HAMA and which allow a sensitive HIV-1 p24 antigen detection.

### Solution to Problem

The present inventors have found:
1) that sandwich immunoassays using human monoclonal antibodies allow a sensitive HIV-1 p24 antigen detection; and
2) that human monoclonal antibodies reduce false positives even when a sample contains HAMA,
and hence the present invention has been achieved.

Thus, the present invention provides:
(1) a reagent for detecting a HIV antigen comprising a first antibody wherein a first human monoclonal antibody recognizing HIV-1 p24 antigen is labeled with a labeling substance,
   a solid phase and
   a second antibody wherein a substance capable of binding to the solid phase is bound to a second human monoclonal antibody recognizing HIV-1 p24 antigen;
(2) the reagent according to (1), which comprises a first reagent containing the first antibody, a second reagent containing particles corresponding to the solid phase and a third reagent containing the second antibody;
(3) the reagent according to (2), wherein the labeling substance is an enzyme and the reagent further comprises a fourth reagent containing a substrate of the enzyme;
(4) the reagent according to any one of (1) to (3), wherein the solid phase carries avidin or streptavidin, and the substance capable of binding to the solid phase is biotin;
(5) the reagent according to any one of (1) to (4), wherein the solid phase is a magnetic particle;
(6) a method for detecting a HIV antigen comprising the steps of:
   forming a complex on a solid phase between HIV-1 p24 antigen contained in a sample, a first antibody wherein a first human monoclonal antibody recognizing HIV-1 p24 antigen is labeled with a labeling substance, and a second antibody wherein a substance capable of binding to the solid phase is bound to a second human monoclonal antibody recognizing HIV-1 p24 antigen, and
   detecting the HIV antigen based on the labeling substance contained in the complex;
(7) the method according to (6), which comprises:
   a step of mixing the antigen with the first antibody,
   a step of mixing the mixture obtained in the previous mixing step, particles corresponding to the solid phase and the second antibody to form the complex on the particles, and
   a step of detecting the HIV antigen based on the labeling substance contained in the complex formed on the particles;
(8) the method according to (6), which comprises:
   a first mixing step in which the antigen and the first antibody are mixed,
   a second mixing step in which the mixture obtained in the first mixing step is mixed with particles corresponding to the solid phase,
   a step of forming the complex on the particle by mixing the mixture obtained in the second mixing step and the second antibody, and
   a step of detecting the HIV antigen based on the labeling substance contained in the complex formed on the particles;
(9) the method according to (6), which comprises:
   a first mixing step in which the antigen and the first antibody are mixed,
   a second mixing step in which the mixture obtained in the first mixing step is mixed with the second antibody,
   a step of forming the complex on particles corresponding to the solid phase by mixing the mixture obtained in the second mixing step and
   the particles, and
   a step of detecting the HIV antigen based on the labeling substance contained in the complex formed on the particles;
(10) the method according to (6), which comprises:
   a first mixing step in which the antigen and the first antibody are mixed,
   a second mixing step in which particles corresponding to the solid phase and the second antibody are mixed,
   a step of forming the complex on the particles by mixing the mixture obtained in the first mixing step and the mixture obtained in the second mixing step, and
   a step of detecting the HIV antigen based on the labeling substance contained in the complex formed on the particles;
(11) the method according to any one of (7) to (10), wherein the labeling substance is an enzyme, the method further comprises a step of mixing a substrate of the enzyme and the complex formed on the particles, and the step of detecting detects the HIV antigen based on the reaction of the enzyme with the substrate;
(12) the method according to (11), wherein the substrate is a luminescent or chromogenic substrate, and the step of detecting detects the HIV antigen by detecting luminescence or color generated by the reaction of the enzyme with the substrate; and
(13) the method according to any one of (6) to (12), wherein the solid phase is a magnetic particle.

### Advantageous Effect of Invention

According to the reagent and method for detecting a HIV antigen according to the present invention, the HIV antigen is detected by using human monoclonal antibodies. Therefore, false positive results can be decreased even when samples contain HAMA. In addition, HIV-1 p24 antigen can be detected with higher sensitivity compared to the conventional methods. Thus, the amount of a sample required for the HIV antigen detection can be decreased. Further, highly precise results can be obtained without any pre-treatment of the sample. Consequently, the complexity of tests may be decreased.

### Brief Description of Drawings

Fig. 1 represents a conceptual drawing of the present reagent for detecting a HIV antigen comprising the first, second and third reagents.
Fig. 2 represents a conceptual drawing of the reaction in the present method for detecting a HIV antigen.

### Best Mode for Carrying out the Invention

The reagent for detecting a HIV antigen according to the present invention comprises a first antibody wherein a first human monoclonal antibody recognizing HIV-1 p24 antigen is labeled with a labeling substance, a solid phase and a second antibody wherein a substance capable of binding to the solid phase is bound to a second human monoclonal antibody recognizing HIV-1 p24 antigen. The reagent for detecting a HIV antigen of the present invention comprises a reagent kit composed of single or multiple reagents. In the reagent for detecting a HIV antigen of the present invention, the first and second antibodies may be contained in separate reagents. Any two or all of the first antibody, the solid phase and the second antibody may be contained in one reagent. The reagent kit may comprise a reagent containing the first antibody and another reagent containing the solid phase and the second antibody. In this case, the second antibody in the second reagent may be bound to the solid phase. The reagent containing all of the first antibody, solid phase and second antibody may also be contemplated. In this case, the second antibody in the reagent may bind to the solid phase.

The reagent for detecting a HIV antigen according to the present invention is preferably a reagent kit in which the first antibody, the solid phase and the second antibody are contained in separate reagents. The solid phase contained in the reagent may include particles described below. Thus, it is preferable that the reagent kit comprises a first reagent containing the first antibody, a second reagent containing the particles corresponding to the solid phase and a third reagent containing the second antibody.

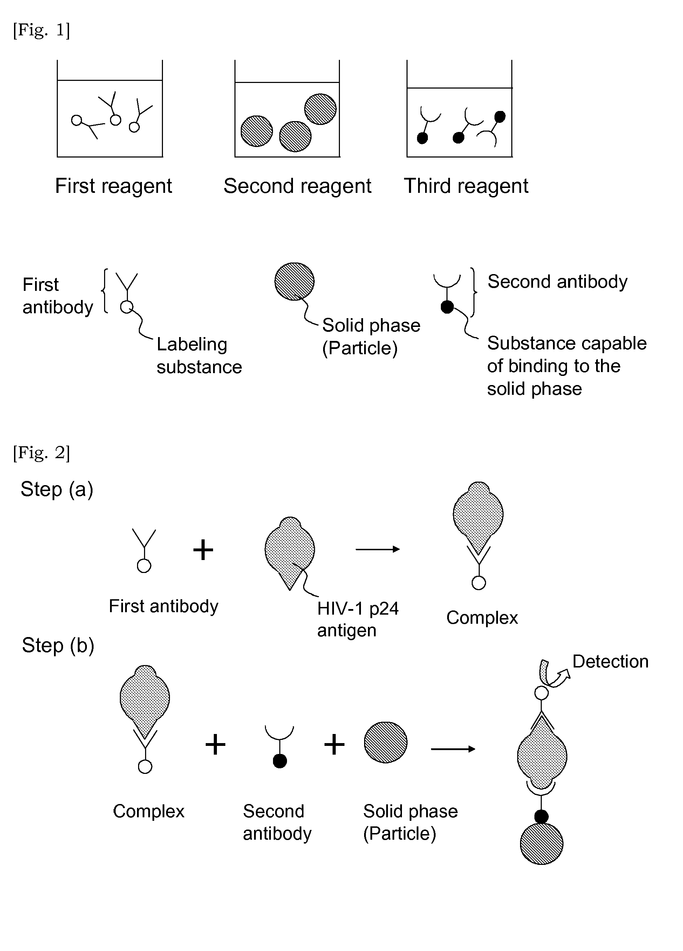

Fig. 1 shows a schematic view of the first, second and third reagents comprised in the reagent kit. The first, second and third reagents contain the first antibody, the particles as the solid phase and the second antibody, respectively.

The first and second human monoclonal antibodies recognizing HIV-1 p24 antigen used in the present invention may be any human monoclonal antibodies recognizing HIV-1 p24 antigen without limitation. The class of the antibodies is not limited and includes IgG, IgM and the like. The antibodies may be fragments of the antibodies and the derivatives thereof provided that they can recognize HIV-1 p24 antigen. The fragments of the antibodies include Fab, F(ab')₂, Fab', sFv and the like fragments. These fragments can be obtained by treating the antibodies according to a well-known method such as the pepsin treatment.

The human monoclonal antibodies recognizing HIV-1 p24 antigen can be obtained by methods well-known to those skilled in the art. For example:
(1) a method comprising fusing lymphocytes of a patient infected with HIV and mouse myeloma cells, selecting a hybridoma producing a desired monoclonal antibody and obtaining the antibody produced from the selected hybridoma;
(2) a method for obtaining a desired antibody by immunizing a mammal that can produce a humanized immunoglobulin (e.g. XENOMOUSE^{®}) with HIV-1 p24 antigen; and
(3) a method comprising transfecting B lymphocytes recovered from blood of a HIV-infected patient with Epstein-Barr virus, screening B lymphocytes for the production of a desired antibody, and introducing a desired antibody producing gene obtained by cloning of the screened B lymphocytes, into a mammalian cell such as CHO (Chinese Hamster Ovary) cell to obtain the desired antibody
   may be mentioned. The antibodies may be commercially available. Specific examples of the antibodies include human monoclonal antibodies recognizing HIV-1 p24 antigen described in Proc. Natl. Acad. Sci. USA, Vol.86, pp.1624-1628, 1989, and human monoclonal antibodies recognizing HIV-1 p24 antigen available from the Belgian company, BioMARIC N.V..

According to the present invention, the first and second human monoclonal antibodies are preferably human monoclonal antibodies obtained from the Belgian company, BioMARIC N.V.

The first and second human monoclonal antibodies are the antibodies which can recognize different parts (epitopes) of the antigen, respectively.

The first antibody is labeled with the detectable labeling substance. The labeling substance may be any labeling substance, without limitation, that can be used in conventional immunoassays. The labeling substance may include, for example, an enzyme, a fluorescent material, a radioisotope and the like.
The enzyme may include, for example, alkaline phosphatase, peroxidase, glucose oxidase, tyrosinase, acid phosphatase and the like. The fluorescent material may include fluorescein isothiocyanate (FITC), green fluorescent protein (GFP), luciferin and the like. The radioisotope may include ¹²⁵I, ¹⁴C, ³²P and the like.
The labeling substance is preferably an enzyme and is more preferably alkaline phosphatase (ALP).

The labeling method of the first human monoclonal antibody with the labeling substance may be any method, without limitation, that is usually used in the art. For example, a method in which the labeling substance is linked to the antibody through a thiol group (-SH group) in the antibody is known. Specifically, the antibody can be labeled with the labeling substance by reacting the labeling substance into which a functional group capable of reacting with a thiol group such as a maleimide group has been introduced, with the first human monoclonal antibody.

The second antibody can bind to the solid phase. The bond between the second antibody and the solid phase is preferably the bond between the substance capable of binding to the solid phase contained in the second antibody (hereinafter also referred to as "the substance for solid phase binding") and a binding substance carried by the solid phase.
The substance for solid phase binding and the binding substance may be any combination of the substances, without limitation, that can specifically bind to each other under the condition of the method for detecting HIV-1 p24 antigen. Such combination may include, for example, biotin and avidin or streptavidin, hapten and anti-hapten antibody, nickel and histidine tag, glutathione and glutathione-S-transferase and the like. The hapten and anti-hapten antibody may include, for example, dinitrophenol (DNP) and anti-DNP antibody.

Preferably, the second antibody and the solid phase are bound via biotin-avidin or biotin-streptavidin linkage. Thus, a preferable combination of the substance for solid phase binding and the binding substance is the combination of biotin and avidin or streptavidin.
The combination of the substance for solid phase binding and the binding substance may be selected from the above-mentioned combinations, without limitation, which can specifically bind to each other. For example, when the substance for solid phase binding comprises biotin, the binding substance is avidin or streptavidin. Conversely, when the substance for solid phase binding comprises avidin or streptavidin, the binding substance is biotin. Preferably, the substance for solid phase binding comprises biotin and the binding substance is avidin or streptavidin. In other words, it is preferred that the second antibody is the antibody which is bound to biotin and the solid phase is the solid phase which carries avidin or streptavidin.

The technique for attaching the substance for solid phase binding to the second human monoclonal antibody to obtain the second antibody capable of binding to the solid phase is known in the art. For example, the second human monoclonal antibody can be attached to the magnetic particle binding substance through a functional group reactive with a primary amino group (-NH₂ group) or a thiol group in the antibody. The functional group reactive with a primary amino group may include succinimide group (NHS group). The functional group reactive with a thiol group may include maleimide group.

The material of the solid phase may be any material used for the solid phase in conventional immunoassays, without limitation. It may include, for example, polymer materials such as latex, rubber, polyethylene, polypropylene, polystyrene, styrene-butadiene copolymers, polyvinyl chloride, polyvinyl acetate, polyacrylamide, polymethacrylate, styrene-methacrylate copolymers, polyglycidyl methacrylate, acrolein-ethylene glycol dimethacrylate copolymers, polyvinylidene difluoride (PVDF), silicone; agarose; gelatin; red blood cell; inorganic materials such as silica gel, glass, inactivated alumina, magnetic body and the like. One or more of these materials may be combined.

The shape of the solid phase may be any shape usually employed for the solid phase in immunoassays, without limitation. For example, it may include microtiter plates, tubes, particles and the like. The solid phase to be contained in the reagent is preferably particles. The particles can be easily included in the reagent. The particles may include a magnetic particle, latex, red blood cell, gelatin particle and the like.

The binding substance may be directly held by the solid phase, or may be held by a substance covering the surface of the solid phase. When the surface of the solid phase has a hydrophilic group such as an amino or carboxyl group, for example, the binding substance may be reacted with the hydrophilic group, so that the solid phase can carry the binding substance. When the surface of the solid phase is covered with a substance such as a polymer or protein, the binding substance may be bound to the substance via chemical bond or intermolecular force, so that the solid phase can carry the binding substance.

The solid phase used in the present invention is preferably a magnetic particle. The magnetic particle is a particle comprising a substrate having magnetic properties. Such magnetic particles are well-known in the art. The known substrate uses, for example, Fe₂O₃ and/or Fe₃O₄, cobalt, nickel, ferrite, magnetite and the like. Preferable magnetic particles may include:
(1) a magnetic particle covered on its surface with a polymer or protein;
(2) a magnetic particle on which surface a carboxyl group is exposed; and
(3) a magnetic particle having a configuration in which a substrate having magnetic properties is dispersed in a resin.

### These magnetic particles can easily carry the above binding substance.

The attachment of the binding substance to the magnetic particle can be carried out by well-known techniques in the art. For example, the techniques based on physical adsorption, covalent bond, ionic bond, the combination thereof may be employed.
For example, when avidin or streptavidin is attached to the magnetic particle, the magnetic particle on which surface a carboxyl group is exposed is reacted chemically to attach avidin or streptavidin to the magnetic particle. More specifically, avidin or streptavidin can be attached to the magnetic particle by reacting an amino group in the molecule of avidin or streptavidin with a carboxyl group exposed on the surface of the particle in the presence of a condensing agent such as water-soluble carboiimide to form an amide bond. When the surface of the magnetic particle is covered with a polymer or protein, avidin or streptavidin can be attached to those substances via chemical bond or intermolecular force, so that avidin or streptavidin is attached to the solid phase. When the magnetic particle has a configuration in which a substrate having magnetic properties is dispersed in a resin, avidin or streptavidin can be attached to the resin via chemical bond or intermolecular force, so that avidin or streptavidin is attached to the solid phase.

The reagent for detecting HIV according to the present invention can comprise other component which does not disturb and/or which promotes the detection of HIV-1 p24 antigen, in addition to the first antibody, the solid phase and the second antibody. The other component may include surfactants, preservatives, serum proteins and the like. For example, each reagent constituting the reagent kit comprising the first reagent containing the first antibody, the second reagent containing the particles as the solid phase and the third reagent containing the second antibody can contain the above other component.

The reagent for detecting HIV according to the present invention may be in liquid state in which the first antibody, the solid phase and the second antibody are suspended or solved in an appropriate solvent together with the above other component. The liquid may be solidified by freeze-drying and the like treatments. Preferably, the reagent for detecting HIV of the present invention is liquid. It is preferable that all of the first, second and third reagents in the reagent kit comprising the first reagent containing the first antibody, the second reagent containing the particles as the solid phase and the third reagent containing the second antibody are liquid.
The appropriate solvent may be any solvent that does not disturb the detection of HIV-1 p24 antigen, without limitation. For example, phosphate buffered saline (PBS), triethanolamine hydrochloride buffer (TEA), Tris-HCl buffer may be used.
The pH of the first, second and third reagents is preferably around 6.0 to 10.0.

Although it may depend on the type of antigens, when the first and second antibodies react with the antigen which may be contained in a sample, the antibodies preferably present in an excess amount to the antigen. Namely, the concentrations of the first and second antibodies upon reaction with the antigen are preferably 0.01 to 10 µg/ml, and more preferably 0.1 to 5 µg/ml.
When the solid phase in the reagent for detecting HIV is a magnetic particle, the reagent for detecting HIV preferably contains 0.1 to 20 mg/ml, more preferably 1 to 10 mg/ml of the magnetic particles.

When the labeling substance which labels the first antibody is an enzyme, the reagent for detecting HIV according to the present invention is preferably a reagent kit which further comprises a reagent containing a substrate of the enzyme. For example, the reagent kit comprising the first reagent containing the first antibody, the second reagent containing the particles as the solid phase and the third reagent containing the second antibody preferably comprises a fourth reagent containing a substrate of the enzyme. The substrate of the enzyme can be a known luminescent substrate such as a chemiluminescent substrate or a bioluminescent substrate and a chromogenic substrate, depending on the enzymes exemplified as the above labeling substance. When the enzyme is alkaline phosphatase, the substrate may include chemiluminescent substrates such as AMPPD^{®} (3-(2'-spiroadamantane)-4-methoxy-4-(3"-phosphoryloxy)phenyl-1,2-dioxetane), CDP-star^{®} (disodium 4-chloro-3-(methoxyspiro{1,2-dioxetane-3,2'-(5'-chloro)tricyclo[3.3.1.1^{3,7}]decan}-4-yl)phenyl phosphate) and CSPD^{®} (disodium 3-(4-methoxyspiro{1,2-dioxetane-3,2-(5'-chloro)tricyclo[3.3.1.1^{3,7}]decan}-4-yl)phenyl phosphate); chromogenic substrates such as p-nitrophenyl phosphate, 5-bromo-4-chloro-3-indolyl-phosphate (BCIP), 4-nitroblue tetrazolium chloride (NBT) and iodo nitro tetrazolium (INT) and the like.

The reagent for detecting HIV according to the present invention can further comprise a washing buffer. For example, the reagent kit comprising the first reagent containing the first antibody, the second reagent containing the particles as the solid phase and the third reagent containing the second antibody preferably comprises the washing buffer as a separate reagent. The washing buffer is preferably a buffer having pH around 6.0 to 10.0, and may include
2-[N-morpholino]ethanesulphonic acid (MES), phosphate buffered saline (PBS) and the like.

The present invention also provides a method for detecting HIV-1 p24 antigen (hereinafter simply referred to as "the detection method").

According to the detection method of the present invention, a complex is formed on the solid phase between HIV-1 p24 antigen contained in the sample, the first antibody wherein the first human monoclonal antibody recognizing HIV-1 p24 antigen is labeled with the labeling substance, and the second antibody wherein the substance capable of binding to the solid phase is bound to the second human monoclonal antibody recognizing HIV-1 p24 antigen; and HIV antigen is detected based on the labeling substance contained in the complex. The detection method of the present invention is not specifically limited so long as the target antigen (HIV-1 p24 antigen) can be detected by using a sandwich method based on the antigen-antibody reaction between the first antibody and the antigen and between the second antibody on the solid phase and the antigen.
When the detection method is carried out with the reagent kit comprising the first reagent containing the first antibody, the second reagent containing the particles as the solid phase and the third reagent containing the second antibody, the sample suspected to contain HIV-1 p24 antigen and the first, second and third reagents may be mixed in any order.

According to the detection method of the present invention, the step of mixing the HIV-1 p24 antigen and the first antibody is preferably carried out first. Due to this step, the antigen is brought into contact with the first antibody to form a complex of HIV-1 p24 and the first antibody. Then, the complex between the HIV-1 p24 antigen, the first antibody and the second antibody is formed on the solid phase by using the second antibody, and HIV antigen is detected based on the labeling substance contained in the complex. The sensitivity of the detection of the target antigen is increased when HIV antigen is detected in this order. The schematic drawing of capturing of the HIV antigen with the first and second antibodies in this order is shown in Fig. 2.

When the reagent kit comprising the first reagent containing the first antibody, the second reagent containing the particles as the solid phase and the third reagent containing the second antibody is used, for example, a preferred detection method comprises:
(a) a step of mixing HIV-1 p24 antigen and the first reagent to form a complex between HIV-1 p24 antigen and the first antibody;
(b) a step of forming the complex formed in the step (a) on the solid phase contained in the second reagent by using the second antibody contained in the third reagent; and
(c) detecting the labeling substance of the first antibody in the complex formed on the particles in the step (b).
This detection method allows a highly sensitive detection of the HIV antigen.

The following modes (1) to (4) may be mentioned as the formation of the complex between HIV-1 p24 antigen, the first antibody and the second antibody on the solid phase using the second antibody, after the step of mixing the antigen and the first antibody.

### (1) To mix the mixture obtained in the mixing step, the particles as the solid phase and the second antibody:

The mixture obtained in the mixing step contains the complex formed of the first antibody and antigen (hereinafter referred to as "first antibody-antigen complex"). In this mode (1), the first antibody-antigen complex is brought into contact with the second antibody and the particles simultaneously. The second antibody binds to the complex to form the complex between the first antibody, the antigen and the second antibody (hereinafter referred to as "first antibody-antigen-second antibody complex") before binding to the particles. Alternatively, the second antibody binds to the particles before binding to the first antibody-antigen complex. As a result, the first antibody-antigen-second antibody complex is formed on the particles.

### (2) To mix the mixture obtained in the mixing step and the particles as the solid phase, and then add the second antibody:

In this mode (2), the first antibody-antigen complex in the mixture is first brought into contact with the particles. The first antibody-antigen complex does not have a binding site with the particles. Thus, the first antibody-antigen complex does not bind to the particles. Then, the second antibody is brought into contact with the antigen-first antibody complex and the particles. Due to this, the second antibody binds to the antigen-first antibody complex to form the first antibody-antigen-second antibody complex before binding to the particles. Alternatively, the second antibody binds to the particles before binding to the first antibody-antigen complex. As a result, the first antibody-antigen-second antibody complex is formed on the particle.

### (3) To mix the mixture obtained in the mixing step and the second antibody, and then the particles as the solid phase are added:

In this mode (3), the first antibody-antigen complex in the mixture first is brought into contact with the second antibody. Due to this, the first antibody-antigen-second antibody complex is formed. Then, the first antibody-antigen-second antibody complex is brought into contact with the particles. Due to this, the first antibody-antigen-second antibody complex binds to the particles. As a result, the first antibody-antigen-second antibody complex is formed on the particles.

### (4) To mix a mixture obtained by mixing the particles as the solid phase and the second antibody, and the mixture obtained in the mixing step:

In this mode (4), the second antibody is brought into contact with the particles. Due to this, the second antibody binds to the particles. Then, the first antibody-antigen complex in the mixture is brought into contact with the second antibody bound to the particles. Due to this, the second antibody bound to the particles binds to the first antibody-antigen complex. As a result, the first antibody-antigen-second antibody complex is formed on the particles. Alternatively, the second antibody which has not been bound to the particles binds to the first antibody-antigen complex to form the first antibody-antigen-second antibody complex, and then the first antibody-antigen-second antibody complex is formed on the particles.

When the detection method is carried out with the reagent kit comprising the first reagent containing the first antibody, the second reagent containing the particles as the solid phase and the third reagent containing the second antibody, the step (b) is carried out by:
(b 1) mixing the complex formed in the step (a) and the second and third reagents;
(b2) mixing the complex formed in the step (a) and the second reagent, and then adding the third reagent;
(b3) mixing the complex formed in the step (a) and the third reagent, and then adding the second reagent; or
(b4) mixing the second and third reagents and then adding the complex formed in the step (a).

The reactions expected in the above respective modes are as follows.

### (b 1) To mix the complex formed in the step (a) and the second and third reagents:

The antigen-first antibody complex formed in the step (a) is brought into contact with the second antibody in the third reagent and the particles in the second reagent simultaneously. Due to this, the second antibody binds to the antigen-first antibody complex to form the first antibody-antigen-second antibody complex before binding to the particles. Alternatively, the second antibody binds to the particle before binding to the antigen-first antibody complex. As a result, the first antibody-antigen-second antibody complex is formed on the particles.

### (b) To mix the complex formed in the step (a) with the second reagent, and then add the third reagent:

The antigen-first antibody complex formed in the step (a) is brought into contact with the particles in the second reagent. In this case, the first antibody or antigen does not have a binding site with the particles. Thus, the antigen-first antibody complex does not bind to the particles. Then, the second antibody in the third reagent is brought into contact with the antigen-first antibody complex and the particles. Due to this, the second antibody binds to the antigen-first antibody complex to form the first antibody-antigen-second antibody complex before binding to the particles. Alternatively, the second antibody binds to the particles before binding to the antigen-first antibody complex. As a result, the first antibody-antigen-second antibody complex is formed on the particles.

### (b3) To mix the complex formed in the step (a) with the third reagent, and then add the second reagent:

The antigen-first antibody complex formed in the step (a) is brought into contact with the second antibody in the third reagent. Due to this, the first antibody-antigen-second antibody complex is formed. Then, the first antibody-antigen-second antibody complex is brought into contact with the particles in the second reagent. As a result, the first antibody-antigen-second antibody complex is formed on the particle.

### (b4) To mix the second and third reagents, and then add the complex formed in the step (a):

By bringing the second antibody in the third reagent into contact with the particle in the second reagent, whole or partial amount of the second antibody bind to the particles. Then, the antigen-first antibody complex formed in the step (a) is mixed with the mixture of the second and third reagents. Due to this, the second antibody bound to the particles binds to the complex to form the first antibody-antigen-second antibody complex on the particles. Alternatively, the second antibody which has not been bound to the particles binds to the first antibody-antigen complex to form the first antibody-antigen-second antibody complex, and then this complex binds to the particles. As a result, the first antibody-antigen-second antibody complex is formed on the particles.

According to the method of the present invention, although it may depend on the type of antigens, the first and second antibodies are preferably present in an excess amount to the antigen contained in the sample. Namely, the concentration of the first and second antibodies upon reaction with the antigen is preferably 0.01 to 10 µg/ml, and more preferably 0.1 to 5 µg/ml. The above reagent for detecting HIV preferably contains the first and second antibodies in the concentrations capable of giving such concentrations of the antibodies.
In case the solid phase is a magnetic particle, it is preferred that the magnetic particles exist in the concentration of 0.1 to 20 mg/ml, more preferably 1 to 10 mg/ml.

The technique for detecting HIV antigen based on the labeling substance may be appropriately selected according to the type of the labeling substance. When the labeling substance is a radioisotope, the technique can be used in which radioactivity is detected by using a known apparatus such as a scintillation counter. When the labeling substance is a fluorescent material, the technique is used in which fluorescence is measured by using a known apparatus such as a luminometer. Preferably, the above labeling substance is an enzyme and luminescence or color is detected which is generated by the reaction of the enzyme with the substrate of the enzyme contained in the fourth reagent. The luminescence or color can be detected by a known apparatus such as a spectrophotometer and luminometer.

When the labeling substance is an enzyme, the detection method further comprises a step of mixing the substrate of the enzyme and the first antibody-antigen-second antibody complex formed on the particles. In the step of detecting, HIV antigen is detected based on the reaction between the enzyme and the substrate. The substrate may include the above luminescent and chromogenic substrates. When the substrate used is the luminescent or chromogenic substrate, HIV antigen can be detected by detecting luminescence or color generated by the reaction of the enzyme and the substrate.

The detection described herein comprises the measurement. The measurement of the labeling substance can be carried out, as the detection of the labeling substance, by using a known apparatus according to the type of the labeling substance. The amount of HIV-1 p24 antigen in the sample can be quantified by using the measured values and a standard curve generated with the solutions containing HIV-1 p24 antigen in known concentrations.

### Examples

The present invention is now described in further detail with referring to the following Examples. However, the present invention is not at all limited to these Examples.
The reagents used in the following Examples 1 to 7 and Comparative Example 1 are prepared as follows.

### (1) Preparation of biotinylated anti-p24 monoclonal antibody

Two kinds of anti-p24 human monoclonal antibodies which recognize different sites were purchased from BioMARIC N.V. (Belgium). One of two obtained anti-p24 human monoclonal antibodies was reacted with biotin-maleimide prepared according to a well-known method to obtain a biotinylated anti-p24 human monoclonal antibody.

### (2) Preparation of biotinylated anti-p24 mouse monoclonal antibody

Anti-p24 mouse monoclonal antibody was purchased from BioMARIC N.V. (Belgium). Anti-p24 mouse monoclonal antibody was reacted with biotin-maleimide prepared according to a well-known method to obtain a biotinylated anti-p24 mouse monoclonal antibody.

### (3) Preparation of alkaline phosphatase (ALP)-labeled anti-p24 human monoclonal antibody

Among two anti-p24 human monoclonal antibodies purchased from BioMARIC N.V. (Belgium) which recognize different sites, the anti-p24 human monoclonal antibody other than the antibody biotinylated in (1) was reacted with ALP-maleimide prepared according to a well-known method to obtain ALP-labeled anti-p24 human monoclonal antibody.

### (4) Preparation of alkaline phosphatase (ALP)-labeled anti-p24 Fab' fragment

The same anti-p24 human monoclonal antibody selected for ALP labeling in (3) was digested with pepsin according to a conventional method to prepare the Fab' fragment. The obtained Fab' fragment was reacted with ALP-maleimide, as described in (3), to obtain ALP-labeled anti-p24 Fab' fragment.

The measurements of HIV-1 p24 antigen were performed by sandwich immunoassays according to the following three measurement procedures.
In the following procedures, antibodies were diluted with an antibody buffer (0.1M MES (2-morpholinoethane sulphonic acid), pH 6.0, 1% bovine serum albumin, 0.15M NaCl and 0.1 % sodium azide).

### Measurement procedure 1

1) The 50 µL of the ALP-labeled anti-p24 monoclonal antibody or ALP-labeled anti p24 Fab' fragment (0.1U/ml) as the first antibody labeled with the labeling substance and 50 µL of HIV-1 p24 antigen solution (concentration of 0 or 325 pg/mL, BioMARIC N.V.) as a sample were placed in a reaction vessel and reacted at 42°C for 2 minutes.
2) The 30 µL of streptavidine-magnetic particles (average particle diameter 2 µm; 5 mg/mL in 20 mM sodium phosphate buffer, pH 7.5) were added and incubated at 42°C for 2.5 minutes.
3) The 50 µL of the biotinylated anti-p24 human monoclonal antibody (0.8 µg/mL) as the second antibody capable of binding to the magnetic particles were added, incubated at 42°C for 2.5 minutes and subjected to a magnetic separation.
4) The 100 to 700 µL of a washing buffer (0.01M MES, pH 6.5 and 0.1% sodium azide) were added, stirred and subjected to a magnetic separation. This washing procedure was repeated for four times in total.
5) The 50 µL of a dispersing buffer (0.01M MES, pH 6.5, 0.1 % sodium azide) were added and stirred to disperse the streptavidin-magnetic particles.
6) The 100 µL of a luminescent substrate solution (CDP-Star, Applied Biosystem) were added, stirred and incubated at 42°C for 5 minutes, and the luminescence intensity was measured with a luminometer.

### Measurement procedure 2

1) The 50 µL of the biotinylated anti-p24 human monoclonal antibody (0.8 µg/mL) as the second antibody capable of binding to the magnetic particles and 50 µL of HIV-1 p24 antigen solution (concentration of 0 or 325 pg/mL) as a sample were placed in a reaction vessel and reacted at 42°C for 2 minutes.
2) The 30 µL of streptavidine-magnetic particles (average particle diameter 2 µm; 5 mg/mL in 20 mM sodium phosphate buffer, pH 7.5) were added and incubated at 42°C for 2.5 minutes.
3) The 50 µL of the ALP-labeled anti-p24 monoclonal antibody or ALP-labeled anti-p24 Fab' fragment (0.1U/ml) as the first antibody labeled with the labeling substance were added, incubated at 42°C for 2.5 minutes and subjected to a magnetic separation.
4) The 100 to 700 µL of the washing buffer were added, stirred and subjected to a magnetic separation. This washing procedure was repeated for four times in total.
5) The 50 µL of the dispersing buffer were added and stirred to disperse the streptavidin-magnetic particles.
6) The 100 µL of a luminescent substrate solution (CDP-Star, Applied Biosystem) were added, stirred and incubated at 42°C for 5 minutes, and the luminescence intensity was measured with a luminometer.

### Measurement procedure 3

1) The 50 µL of the biotinylated anti-p24 monoclonal antibody (0.8 µg/mL) as the second antibody capable of binding to the magnetic particles and 50 µL of HIV-1 p24 antigen solution (concentration of 0 or 325 pg/mL) as a sample were placed in a reaction vessel and reacted at 42°C for 2 minutes.
2) The 30 µL of streptavidine-magnetic particles (average particle diameter 2 µm; 5 mg/mL in 20 mM sodium phosphate buffer, pH 7.5) were added, incubated at 42°C for 2.5 minutes and then subjected to a magnetic separation.
3) The 100 to 700 µL of the washing buffer were added, stirred and subjected to a magnetic separation. This washing procedure was repeated for four times in total.
4) The 50 µL of the ALP-labeled anti-p24 monoclonal antibody or ALP-labeled anti-p24 Fab' fragment (0.1U/ml) as the first antibody labeled with the labeling substance were added, incubated at 42°C for 2.5 minutes and subjected to a magnetic separation.
5) The 100 to 700 µL of the washing buffer were added, stirred and subjected to a magnetic separation. This washing procedure was repeated for four times in total.
6) The 50 µL of the dispersing buffer were added and stirred to disperse the streptavidin-magnetic particles.
7) The 100 µL of a luminescent substrate solution (CDP-Star, Applied Biosystem) were added, stirred and incubated at 42°C for 5 minutes, and the luminescence intensity was measured with a luminometer.

### Example 1

The measurement was carried out according to Measurement Procedure 1 with the biotinylated anti-p24 antigen human monoclonal antibody (0.8 µg/mL) prepared in (1) and the ALP-labeled anti-p24 antigen human monoclonal antibody prepared in (3).

### Comparative Example 1

The measurement was carried out according to Measurement Procedure 1 with the biotinylated anti-p24 mouse monoclonal antibody (0.8, 4.0 and 8.0 µg/mL) prepared in (2), instead of the biotinylated anti-p24 antibody human monoclonal antibody used in Example 1.

### Based on the results obtained in the above Example 1 and

Comparative Example 1, ratios of the results (specific signals) obtained by using a sample containing HIV-1 p24 antigen (antigen solution) and the results (non-specific signals) obtained with using a sample without HIV-1 p24 antigen (signal/noise ratios; S/N ratios) were calculated. The results are summarized in Table 1.

**[Table 1]**

| | Comparative Example 1 | | | Example 1 |
|---|---|---|---|---|
| p24 antigen concentration (pg/mL) | Antibody amount (µg/mL) | | | |
| | 8 | 4 | 0.8 | 0.8 |
| | Luminescence intensity | Luminescence intensity | Luminescence intensity | Luminescence intensity |
| 0 | 2,044 | 2,025 | 2,050 | 2,387 |
| 325 | 320,192 | 643,855 | 232,617 | 1,220,458 |
| S/N ratio | 157 | 318 | 113 | 511 |

The above results show that when anti-p24 human monoclonal antibodies were used according to the present invention, the S/N ratio and the specific signal in the HIV-1 p24 antigen measurements are nearly 5 times higher than anti-p24 mouse monoclonal antibody at the same amount of the antibodies used (0.8 µg/mL). Further, the highest specific signal with anti-p24 mouse monoclonal antibody was less than the half of the specific signal with anti-p24 human monoclonal antibody, indicating that anti-p24 human monoclonal antibodies allow a highly sensitive detection of HIV-1 p24 antigen.

### Example 2

The measurement was carried out according to Measurement Procedure 1 with the same antibodies as Example 1, i.e. with the biotinylated anti-p24 human monoclonal antibody prepared in (1) and the ALP-labeled anti-p24 human monoclonal antibody (hereinafter referred to as "IgG-ALP") prepared in (3).

### Example 3

The measurement was carried out according to Measurement Procedure 2 with the same antibodies used in Example 2.

### Example 4

The measurement was carried out according to Measurement Procedure 3 with the same antibodies used in Example 2.

### Example 5

The measurement was carried out according to Measurement Procedure 1 with the ALP-labeled anti-p24 Fab' fragment (Fab'-ALP) prepared in (4), instead of the ALP-labeled anti-p24 human monoclonal antibody used in Example 2.

### Example 6

The measurement was carried out according to Measurement Procedure 2 with the same antibodies used in Example 5.

### Example 7

The measurement was carried out according to Measurement Procedure 3 with the same antibodies used in Example 5.

Based on the results obtained in the above Examples 2 to 7, ratios of the results (specific signals) obtained by using a sample containing HIV-1 p24 antigen (antigen solution) and the results (non-specific signals) obtained with using a sample without HIV-1 p24 antigen (signal/noise ratios; S/N ratios) were calculated. The results are summarized in Tables 2 and 3.

**[Table 2]**

| p24 antigen concentration (pg/mL) | Example 2 | Example 3 | Example 4 |
|---|---|---|---|
| | Measurement Procedure 1 | Measurement Procedure 2 | Measurement Procedure 3 |
| | Luminescence intensity | Luminescence intensity | Luminescence intensity |
| 0 | 3,741 | 5,013 | 14,527 |
| 325 | 1,935,155 | 326,865 | 567,524 |
| S/N | 517 | 65 | 39 |

**[Table 3]**

| p24 antigen concentration (pg/mL) | Example 5 | Example 6 | Example 7 |
|---|---|---|---|
| | Measurement Procedure 1 | Measurement Procedure 2 | Measurement Procedure 3 |
| | Luminescence intensity | Luminescence intensity | Luminescence intensity |
| 0 | 7,030 | 5,103 | 7,494 |
| 325 | 2,688,389 | 816,209 | 1,281,416 |
| S/N | 382 | 160 | 171 |

The above results show that when anti-p24 human monoclonal antibodies were used according to the present invention, specific signals can be detected regardless of the order of reactions of Measurement Procedures 1 to 3 and regardless of whether the antibody is IgG-ALP or Fab'-ALP. Among these, it is also found that the S/N ratio is higher when Measurement Procedure 1 is used, and that the sensitivity of the antigen detection is higher when IgG-ALP is used than Fab'-ALP is used.

### Example 8 and Comparative Example 2

The detection of HIV-1 p24 antigen was carried out according to Measurement Procedure 1 with the ALP-labeled anti-p24 antigen human monoclonal antibody prepared in (4) as the first antibody, the biotinylated anti-p24 antigen human monoclonal antibody (0.8 µg/mL) prepared in (1) as the second antibody and a commercial HIV seroconversion panel sample, HIV-1 Seroconversion Panel BB (PRB952) (BBI Diagnostics) as a sample.

The results of the HIV antigen detection according to Example 8 are shown in Table 4. As Comparative Example 2, the results of the measurements of the above HIV seroconversion panel sample with Lumipulse^{®} I HIV-1 p24 (Fujirebio Inc.), a commercial reagent for detecting HIV-1 p24 antigen, which are described in the document attached to Lumipulse^{®} HIV-1 p24 are shown. Lumipulse^{®} I HIV-1 p24 uses anti-p24 antigen mouse monoclonal antibodies as the antibodes for detecting HIV-1 p24 antigen. Lumipulse^{®} I HIV-1 p24 requires a pre-treatment step of a sample in which a sample treatment solution containing a surfactant, polyoxyethylene octylphenyl ether, is added to the sample and stirred.

**[Table 4]**

| Sample No. | Days after initial blood taking | Example 8 | Comparative Example 2 |
|---|---|---|---|
| PRB952-01 | 0 | - | - |
| PRB952-02 | 7 | + | - |
| PRB952-03 | 10 | + | + |
| PRB952-04 | 14 | + | + |
| PRB952-05 | 17 | + | + |
| PRB952-06 | 21 | - | - |

As can be seen from Table 4, HIV-1 p24 antigen was detected with Lumipulse^{®} I HIV-1 p24 after 10 days from the initial blood taking. Contrary to this, HIV-1 p24 antigen can be detected after 7 days from the initial blood taking in the HIV antigen detection with anti-p24 human monoclonal antibodies according to the present invention. Thus, the HIV antigen detection with anti-p24 human monoclonal antibody according to the present invention allows the detection of HIV-1 p24 antigen three days earlier than Lumipulse^{®} HIV-1 p24. It is also found that according to the HIV antigen detection of the present invention, HIV-1 p24 antigen can be detected with high sensitivity without pre-treatment of a sample as required in Lumipulse^{®} I HIV-1 p24.

The present application relates to Japanese Patent Application No. 2007-34037 filed on December 28, 2007, whose claims, specification, drawings and abstract are incorporated herein by reference.

## Claims

1. A reagent for detecting a HIV antigen comprising
a first antibody wherein a first human monoclonal antibody recognizing HIV-1 p24 antigen is labeled with a labeling substance,
a solid phase and
a second antibody wherein a substance capable of binding to the solid phase is bound to a second human monoclonal antibody recognizing HIV-1 p24 antigen.

2. The reagent according to Claim 1, which comprises a first reagent containing the first antibody, a second reagent containing particles corresponding to the solid phase and a third reagent containing the second antibody.

3. The reagent according to Claim 2, wherein the labeling substance is an enzyme and the reagent further comprises a fourth reagent containing a substrate of the enzyme.

4. The reagent according to Claim 1, wherein the solid phase carries avidin or streptavidin, and the substance capable of binding to the solid phase is biotin.

5. The reagent according to Claim 1, wherein the solid phase is a magnetic particle.

6. A method for detecting a HIV antigen comprising the steps of:
forming a complex on a solid phase between HIV-1 p24 antigen contained in a sample, a first antibody wherein a first human monoclonal antibody recognizing HIV-1 p24 antigen is labeled with a labeling substance, and a second antibody wherein a substance capable of binding to the solid phase is bound to a second human monoclonal antibody recognizing HIV-1 p24 antigen, and
detecting the HIV antigen based on the labeling substance contained in the complex.

7. The method according to Claim 6, which comprises:
a step of mixing the antigen and the first antibody,
a step of mixing the mixture obtained in the previous mixing step, particles corresponding to the solid phase and the second antibody to form the complex on the particles, and
detecting the HIV antigen based on the labeling substance contained in the complex formed on the particles.

8. The method according to Claim 6, which comprises:
a first mixing step in which the antigen and the first antibody are mixed,
a second mixing step in which the mixture obtained in the first mixing step is mixed with particles corresponding to the solid phase,
a step of forming the complex on the particles by mixing the mixture obtained in the second mixing step and the second antibody, and
a step of detecting the HIV antigen based on the labeling substance contained in the complex formed on the particles.

9. The method according to Claim 6, which comprises:
a first mixing step in which the antigen and the first antibody are mixed,
a second mixing step in which the mixture obtained in the first mixing step is mixed with the second antibody,
a step of forming the complex on particles corresponding to the solid phase by mixing the mixture obtained in the second mixing step and the particles, and
a step of detecting the HIV antigen based on the labeling substance contained in the complex formed on the particles.

10. The method according to Claim 6, which comprises:
a first mixing step in which the antigen and the first antibody are mixed,
a second mixing step in which particles corresponding to the solid phase and the second antibody are mixed,
a step of forming the complex on the particles by mixing the mixture obtained in the first mixing step and the mixture obtained in the second mixing step, and
a step of detecting the HIV antigen based on the labeling substance contained in the complex formed on the particles.

11. The method according to Claim 7, wherein the labeling substance is an enzyme, the method further comprises a step of mixing a substrate of the enzyme and the complex formed on the particles, and the step of detecting detects the HIV antigen based on the reaction of the enzyme with the substrate.

12. The method according to Claim 11, wherein the substrate is a luminescent or chromogenic substrate, and the step of detecting detects the HIV antigen by detecting luminescence or color generated by the reaction of the enzyme with the substrate.

13. The method according to Claim 6, wherein the solid phase is a magnetic particle.
